# FASCICULE DE BREVET EUROPEEN

(11) **EP 1 799 067 B1**
(45) Date de publication et mention de la délivrance du brevet: **17.08.2016**
(21) Numéro de dépôt: 05790048.2
(22) Date de dépôt: 08.07.2005
(51) Int. Cl.: A45D 33/00, A45D 34/00, A45D 34/04, A45D 29/00

(54) **PROCEDE DE MAQUILLAGE AU MOYEN D'UNE COMPOSITION MAGNETIQUE COMPORTANT AU MOINS UN PIGMENT DIFFRACTANT**
MAKEUP-VERFAHREN UNTER VERWENDUNG EINER MAGNETISCHEN ZUSAMMENSETZUNG MIT MINDESTENS EINEM DIFFRAKTIVEM PIGMENT
MAKE-UP METHOD USING A MAGNETIC COMPOSITION CONTAINING AT LEAST ONE DIFFRACTIVE PIGMENT

(30) Priorité: 05.10.2004 FR 0410501; 20.10.2004 US 619928 P
(43) Date de publication de la demande: 27.06.2007
(73) Titulaire: L'Oréal, 75008 Paris (FR)
(72) Inventeur: THEVENET, Ludovic, F-92340 BOURG LA REINE (FR)
(74) Mandataire: Nony
(86) Numéro de dépôt international: PCT/FR2005/050559
(87) Numéro de publication internationale: WO 2006/037902

(56) Documents cités:
- EP-A- 1 264 562
- WO-A-02/28356
- WO-A2-2004/007096
- US-A1- 2003 031 870

## Description

La présente invention concerne un procédé de maquillage d'un support, naturel tel que la peau, les phanères ou les lèvres, ou artificiel tel que de faux ongles, ainsi qu'un kit pour la mise en oeuvre de ce procédé.

Il existe un besoin pour bénéficier de nouveaux effets esthétiques dans le domaine du maquillage et l'invention vise à répondre à ce besoin.

EP1264562 divulgue un applicateur magnétique associé à un produit qui peut comporter des fibres ou paillettes magnétiques.

WO02/28356 décrit une composition comportant des paillettes interférentielles.

### PROCEDE DE MAQUILLAGE

Selon l'un de ses aspects, l'invention a pour objet un procédé de maquillage de matières kératiniques, notamment la peau, les lèvres ou les phanères, comportant les étapes suivantes :
- déposer sur le support à maquiller au moyen d'un applicateur cosmétique non magnétique au moins une composition cosmétique,
   cette composition cosmétique comportant :
   - au moins un corps présentant une susceptibilité magnétique non nulle et mobile sous l'effet d'un champ magnétique,
   - au moins un pigment diffractant,
- exposer au moins partiellement la composition à un champ magnétique de manière à modifier l'orientation et/ou déplacer au moins une partie des corps magnétiques, le champ magnétique étant appliqué de manière à former au moins un motif sur la composition.

Les corps magnétiques peuvent être différents du pigment diffractant ou en variante le pigment diffractant peut constituer tout ou partie des corps magnétiques.

Lorsque les corps magnétiques apportent de la couleur, une modification de leur orientation sous l'effet du champ magnétique peut conduire à une modification de l'aspect de la composition.

Lorsque les corps magnétiques sont déplacés, la forme du dépôt de la composition peut s'en trouver affectée, ce qui permet de créer un relief par exemple.

Lors de l'application de la composition, celle-ci peut être non exposée au champ magnétique. Ce dernier peut être exercé une fois l'application effectuée.

Le champ magnétique peut être appliqué de manière à former au moins un motif sur la composition, celui-ci étant par exemple lié à la géométrie des lignes de champ.

L'invention rend ainsi possible de créer de nouveaux effets de maquillage avec une composition cosmétique, en permettant par exemple de réaliser des motifs en relief ou conférant une impression de relief ou divers autres motifs, géométriques ou non.

Le champ magnétique peut encore être appliqué de manière à modeler la clarté et/ou la couleur d'une région au moins du visage ou du corps sur laquelle la composition a été appliquée.

Par exemple, quand la composition cosmétique est un fond de teint, l'orientation des corps magnétiques sous l'effet du champ magnétique rend possible de modifier la clarté de la composition et de modeler ainsi l'aspect du visage selon les régions exposées au champ magnétique, afin notamment de réaliser un maquillage du type clair/obscur, sans transition brutale si on le souhaite entre les régions claires et les régions obscures. Le champ magnétique peut par exemple être appliqué de manière à assombrir les côtés du visage afin de le faire paraître plus mince qu'il n'est réellement.

Dans un exemple de mise en oeuvre de l'invention, une couche d'une deuxième composition cosmétique est appliquée sur la première, contenant les corps magnétiques, par exemple en vue d'obtenir un effet de profondeur, de brillance, de lissage ou autre. Cette deuxième composition peut être transparente, colorée ou non. La deuxième composition est par exemple destinée à l'application sur les lèvres ou les ongles. La deuxième composition peut encore être appliquée sur le support avant la première composition, par exemple pour créer un fond coloré ou améliorer la tenue de la première composition et/ou le confort.

Le champ magnétique peut être appliqué jusqu'à obtenir un aspect figé de la composition contenant les corps magnétiques, c'est-à-dire que l'aspect de celle-ci cesse d'évoluer même si le champ magnétique perdure. En variante, le champ magnétique peut être appliqué pendant une durée inférieure à celle provoquant l'orientation et/ou le déplacement définitif de la totalité des corps magnétiques de la région exposée.

Lorsque la clarté et/ou la couleur de la première composition changent progressivement sous l'effet du champ magnétique, l'utilisateur peut arrêter de soumettre les corps magnétiques au champ lorsque la composition présente l'aspect souhaité.

Le champ magnétique peut être exercé successivement sur différentes régions du support revêtues de la composition.

Le champ magnétique peut être exercé sur des régions disjointes du support, afin par exemple de créer des motifs séparés.

Une région du support revêtue de la composition peut être non exposée au champ magnétique, de manière à ne pas modifier dans cette région l'aspect de la composition après son dépôt.

Deux régions du support peuvent être exposées de manière inégale au champ magnétique.

La composition peut être appliquée de diverses manières au moyen d'un applicateur cosmétique non magnétique, choisi par exemple parmi les pinceaux, les embouts floqués et les mousses, les tissés, les non-tissés, les brosses ou peignes.

Dans un exemple de mise en oeuvre de l'invention, la composition est appliquée sur le support au travers d'un masque ajouré. Cela peut permettre par exemple de réaliser un motif prédéterminé, correspondant à la forme de l'ajour. Au moins une région du support recouverte par la composition peut ensuite être exposée au champ magnétique.

La composition peut prendre un état empêchant tout nouveau changement d'orientation des corps magnétiques sous l'effet d'un champ magnétique après une durée de séchage donnée. C'est par exemple le cas d'un vernis à ongles. L'orientation des corps magnétiques peut encore, dans certains cas, être modifiée à tout moment, notamment lorsque la première composition ne sèche pas ou présente une durée de séchage très longue. Cela peut être le cas par exemple d'un fond de teint.

Les corps magnétiques peuvent se présenter sous diverses formes.

### CORPS MAGNETIQUES

L'expression « corps magnétiques » ne doit pas être comprise de manière limitative et couvre des particules, fibres ou agglomérats de particules et/ou de fibres, de toutes formes, présentant une susceptibilité magnétique non nulle.

La concentration en corps magnétiques dans la composition est par exemple comprise entre environ 0,05 % et environ 50 % en masse, notamment entre environ 0,1 % et environ 40 % en masse, mieux entre environ 1 % et environ 30 % en masse.

La composition appliquée peut comporter des fibres magnétiques ou autres corps asphériques, tels que des chaînes de particules ou de fibres.

De préférence, les corps magnétiques ne présentent pas d'aimantation rémanente en l'absence de champ magnétique.

Les corps magnétiques peuvent comporter tout matériau magnétique présentant une sensibilité aux lignes d'un champ magnétique, que ce champ soit produit par un aimant permanent ou issu d'une induction, ce matériau étant par exemple choisi parmi le nickel, le cobalt, le fer, leurs alliages et oxydes, notamment Fe₃O₄, et aussi le gadolinium, le terbium, le dysprosium, l'erbium, leurs alliages et oxydes. Le matériau magnétique peut être de type « doux » ou « dur ». Le matériau magnétique peut notamment être du fer doux.

Les corps magnétiques peuvent présenter ou non une structure multicouche, comportant au moins une couche d'un matériau magnétique, tel que par exemple le fer, le nickel, le cobalt, leurs alliages et oxydes, notamment Fe₃O₄.

Les corps magnétiques sont de préférence asphériques, présentant par exemple une forme allongée. Ainsi, lorsque ces corps sont soumis au champ magnétique, ils tendent à s'orienter avec leur axe longitudinal dans l'alignement des lignes de champ, et subissent un changement d'orientation qui se traduit par un changement d'aspect de la composition.

Lorsque les corps magnétiques sont des particules sensiblement sphériques, de préférence leur aspect est inhomogène, de manière à ce qu'un changement d'orientation induise un changement d'aspect.

La dimension des corps, quelle que soit leur forme, est par exemple comprise entre 1 nm et 10 mm, mieux entre 10 nm et 5 mm, mieux encore entre 100 nm et 1 mm, par exemple entre 0,5 µm et 300 µm ou 1 µm et 150 µm. La dimension est celle donnée par la distribution statistique à la moitié de la population, dite D50.

Lorsque les corps sont des particules n'ayant pas une forme allongée ou ayant une forme allongée avec un facteur de forme assez faible, la dimension des particules est par exemple inférieure à 1 mm.

Les corps magnétiques sont par exemple des pigments magnétiques.

### Pigments magnétiques

Des pigments convenant tout particulièrement sont les nacres comportant de l'oxyde de fer Fe₃O₄. Des pigments présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

A titre encore d'exemple de pigment magnétique susceptible d'entrer dans la formulation de la composition, on peut citer les particules d'oxyde de fer noir, par exemple celles commercialisées sous la dénomination SICOVIT noir E172 par la société BASF.

Les pigments magnétiques peuvent encore comporter du fer métal, notamment du fer doux passivé, par exemple obtenu à partir de fer carbonyle en mettant en oeuvre le procédé décrit dans le brevet US 6 589 331, dont le contenu est incorporé par référence. Ces particules peuvent comporter une couche d'un oxyde de surface.

Des particules à base de fer doux sont commercialisées notamment sous la dénomination STAPA^{®} WM IRON VP 041040 par la société ECKART.

### Fibres magnétiques

Le terme « fibres » désigne des corps généralement allongés, présentant par exemple un facteur de forme allant de 3,5 à 2 500 ou de 5 à 500, par exemple de 5 à 150. Le facteur de forme est défini par le rapport L/D, où L est la longueur de la fibre et D le diamètre du cercle dans lequel s'inscrit la plus grande section transversale de la fibre.

La section transversale des fibres peut s'inscrire par exemple dans un cercle de diamètre allant de 2 nm à 500 µm, par exemple allant de 100 nm à 100 µm, voire de 1 µm à 50 µm.

Les fibres peuvent présenter par exemple une longueur allant de 1 µm à 10 mm, par exemple de 0,1 mm à 5 mm, voire de 0,3 mm à 3,5 mm.

Les fibres peuvent présenter une masse allant par exemple de 0,15 à 30 deniers (masse en gramme pour 9 km de fil), par exemple de 0,18 à 18 deniers.

La forme en section transversale des fibres peut être quelconque, par exemple circulaire ou polygonale, notamment carrée, hexagonale ou octogonale.

La composition peut comporter des fibres pleines ou creuses, indépendantes ou liées entre elles, par exemple tressées.

La composition peut comporter des fibres ayant des extrémités épointées et/ou arrondies, par exemple par polissage.

Les fibres peuvent ne pas voir leur forme sensiblement modifiée lorsqu'elles sont introduites dans la composition, étant par exemple initialement rectilignes et suffisamment rigides pour conserver leur forme. En variante, les fibres peuvent présenter une souplesse leur permettant de se déformer sensiblement au sein de la composition.

Les fibres peuvent comporter une teneur non nulle, pouvant aller jusqu'à 100 %, d'un matériau magnétique choisi parmi les matériaux magnétiques doux, les matériaux magnétiques durs, notamment à base de fer, de zinc, de nickel, de cobalt ou de manganèse et leurs alliages et oxydes, notamment Fe₃O₄, les terres rares, le sulfate de baryum, les alliages de fer silicium, éventuellement chargés en molybdène, Cu₂MnAl, MnBi, ou un mélange de ceux-ci, cette liste n'étant pas limitative.

Lorsque la composition comporte des fibres contenant des particules magnétiques, ces dernières peuvent être présentes par exemple au moins à la surface de la fibre, voire à la surface des fibres uniquement, à l'intérieur de la fibre uniquement ou encore être dispersées au sein de la fibre de manière sensiblement homogène.

Les fibres peuvent comporter par exemple un coeur non magnétique avec une pluralité de particules magnétiques à sa surface.

Les fibres peuvent encore comporter une matrice synthétique contenant une pluralité de grains magnétiques dispersés en son sein.

Le cas échéant, une matière synthétique chargée de particules magnétiques peut elle-même être enrobée par une écorce non magnétique. Une telle écorce constitue par exemple une barrière isolant le ou les matériaux magnétiques du milieu ambiant et/ou peut amener de la couleur. Les fibres peuvent comporter un coeur magnétique monolithique et être enrobées par une écorce non magnétique, ou cela peut être l'inverse.

La composition peut comporter des fibres réalisées par extrusion ou co-extrusion d'une ou plusieurs matières polymériques, notamment thermoplastiques et/ou élastomères. L'une des matières extrudées peut contenir une charge de particules magnétiques dispersées.

La fibre peut comporter une matière synthétique choisie parmi les polyamides, PET, acétates, polyoléfines, notamment PE ou PP, PVC, polyester bloc amide, Rilsan^{®} plastifié, élastomères, notamment élastomères de polyester, élastomères de PE, élastomères de silicone, élastomères de nitrile ou un mélange de ces matériaux, cette liste n'étant pas limitative.

La composition peut contenir des fibres composites comportant un coeur magnétique enrobé au moins partiellement par au moins un matériau amagnétique, synthétique ou naturel. L'enrobage du coeur magnétique peut se faire par exemple par co-extrusion, autour du coeur, d'une écorce en un matériau non magnétique.

L'enrobage du coeur peut encore s'effectuer autrement, par exemple par polymérisation *in situ.*

Le coeur peut être monolithique ou comporter une charge de grains magnétiques dispersés dans une matrice.

La composition peut encore contenir des fibres composites obtenues par enrobage par une matière synthétique, chargée de particules magnétiques, d'un coeur amagnétique, synthétique ou naturel, le coeur étant composé par exemple d'une fibre de bois, de rayonne, de polyamide, d'une matière végétale, de polyoléfine, notamment de polyéthylène, de Nylon^{®}, de polyimide-amide, d'aramide, cette liste n'étant pas limitative.

La composition peut encore comporter des particules composites magnétiques, notamment un latex magnétique.

### Particules composites magnétiques

Une particule composite magnétique est un matériau composite constitué d'une matrice organique ou minérale et de grains magnétiques. Les particules composites magnétiques peuvent ainsi comporter à leur surface et/ou en leur sein des grains d'un matériau magnétique. Les particules composites peuvent être constituées d'un coeur magnétique enrobé d'une matrice organique ou minérale, ou inversement.

Les particules composites magnétiques comportent par exemple l'un des matériaux magnétiques précités.

La dimension des particules composites magnétiques est par exemple comprise entre 1 nm et 1 mm, mieux entre 100 nm et 500 µm, mieux encore entre 500 nm et 100 µm. Par « dimension », on désigne la dimension donnée par la distribution granulométrique statistique à la moitié de la population, dite D50.

La thèse de C. GOUBAULT, 23 Mars 2004, incorporée ici par référence, rappelle au chapitre 1 l'état de l'art en matière de particules composites magnétiques, et dresse une liste de procédés de préparation pouvant être utilisés pour préparer des particules composites magnétiques, à savoir une synthèse séparée des grains magnétiques et de la matrice, une synthèse des grains magnétiques au contact de la matrice ou une synthèse de la matrice en présence des grains magnétiques.

La société KISKER commercialise des particules magnétiques composites à matrice minérale, composée de silice. Les sociétés DYNAL, SERADYN, ESTAPOR et ADEMTECH proposent des particules magnétiques composites à matrice organique, susceptibles également d'être utilisées dans l'invention.

Plus particulièrement, la société ESTAPOR commercialise sous la référence M1-070/60 des latex magnétiques constitués de grains de ferrite uniformément répartis dans une matrice polystyrène, ce latex comportant 65 % d'oxyde de fer, le diamètre moyen des particules de polystyrène étant de 890 nm et la teneur massique en matières sèches de 10%.

### Ferrofluide

La composition peut comporter un ferrofluide, c'est-à-dire une suspension colloïdale stable de particules magnétiques, notamment de nanoparticules magnétiques.

Les particules, d'une taille par exemple de l'ordre de quelques dizaines de nanomètres, sont dispersées dans un solvant (eau, huile, solvant organique), soit à l'aide d'un tensioactif ou d'un agent dispersant, soit par des interactions électrostatiques.

Les ferrofluides sont par exemple préparés par broyage de ferrites ou autres particules magnétiques jusqu'à l'obtention de nanoparticules qui sont ensuite dispersées dans un fluide contenant un surfactant, lequel s'adsorbe sur les particules et les stabilise, ou par précipitation en milieu basique d'une solution d'ions métalliques.

Chaque particule du ferrofluide présente un moment magnétique déterminé par la taille de la particule et par la nature du matériau magnétique.

Sous l'action d'un champ magnétique, les moments magnétiques des particules tendent à s'aligner suivant les lignes de champ, avec apparition d'une aimantation non nulle dans le liquide. Si le champ est annulé, il n'y a pas d'hystérésis et l'aimantation s'annule.

Au-delà d'une valeur seuil de champ, on peut également provoquer des changements macroscopiques dans le liquide, par exemple l'apparition de pics ou une modification des propriétés rhéologiques.

La dénomination « ferrofluide » englobe également une émulsion de gouttelettes de ferrofluide dans un solvant. Chaque goutte contient alors des particules magnétiques colloïdales en suspension stable. Cela permet de disposer d'un ferrofluide dans tout type de solvant. La dimension des particules magnétiques en suspension dans le ferrofluide est par exemple comprise entre 1 nm et 10 µm, mieux entre 1 nm et 1 µm, mieux encore entre 1 nm et 100 nm. Par « dimension », on désigne la dimension donnée par la distribution granulométrique statistique à la moitié de la population, dite D50.

On peut citer notamment les ferrofluides commercialisés par la société LIQUIDS RESEARCH LTD sous les références :
- WHKS1S9 (A, B ou C), qui est un ferrofluide à base aqueuse comportant de la magnetite (Fe₃O₄), ayant des particules de 10 nm de diamètre.
- WHJS1 (A, B ou C), qui est un ferrofluide à base d'iso-paraffine et de particules de magnetite (Fe₃O₄) de 10 nm de diamètre.
- BKS25_dextran, qui est un ferrofluide à base aqueuse stabilisé par du dextran, comportant des particules de magnetite (Fe₃O₄) de 9 nm de diamètre.

### Chaînes de particules et/ou de fibres magnétiques

La composition peut comporter des agglomérats de particules ou fibres dont la plus grande dimension, par exemple la longueur, est par exemple comprise entre 1 nm et 10 mm, par exemple entre 10 nm et 5 mm, ou entre 100 nm et 1 mm, ou encore entre 0,5 µm et 3,5 mm, par exemple entre 1 µm et 150 µm. La dimension désigne celle donnée par la distribution granulométrique statistique à la moitié de la population, dite D50.

Des chaînes de particules magnétiques peuvent être obtenues par exemple en assemblant des particules magnétiques colloïdales, comme cela est décrit dans les publications « Permanently linked monodisperse paramagnetic chains », E.M. Furst, C. Suzuki, M. Fermigier, A.P. Gast, Langmuir, 14, 7334-7336 (1998), « Suspensions de particules magnétiques », M. Fermigier, Y. Grasselli, Bulletin de la SFP (105) juillet 96, et « Flexible magnetic filaments as micromechanical sensors », C. Goubault, P. Jop, M. Fermigier, J. Baudry, E. Bertrand, J. Bibette, Phys. Rev. Lett., 91, 26, 260802-1 à 260802-4 (2003), dont les contenus sont incorporés par référence.

Il est notamment décrit dans ces articles comment procéder pour obtenir des chaînes de particules de latex magnétiques comportant une matrice de polystyrène contenant des grains d'oxyde de fer et fonctionnalisées en surface, liées entre elles de façon permanente suite à une réaction chimique, notamment des liaisons covalentes entre les surfaces des particules adjacentes ; il est également décrit un procédé d'obtention de chaînes de gouttelettes d'émulsion de ferrofluides, liées entre elles par interactions de nature physique. La longueur ainsi que le diamètre des chaînes permanentes ainsi obtenues peuvent être contrôlés. De telles chaînes magnétiques constituent des objets magnétiques anisotropes orientables et déplaçables sous l'effet d'un champ magnétique.

Les dimensions des chaînes magnétiques peuvent répondre aux mêmes conditions que les fibres magnétiques.

Selon un aspect de l'invention, la composition comporte au moins un pigment diffractant.

### PIGMENT DIFFRACTANT

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière. Un tel pigment est encore parfois appelé pigment holographique ou à effet arc en ciel.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002, dont le contenu est incorporé par référence.

Le pigment diffractant peut être réalisé avec des motifs ayant différents profils, notamment triangulaires, symétriques ou non, en créneaux, de largeur constante ou non, sinusoïdaux, en escalier.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non, de même linéature ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AlN, BN, B₄C, WC, TiC, TiN, N₄Si₃, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de SiO₂, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : MgF₂/Al/MgF₂, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du MgF₂ peut être comprise entre 80 et 95 % du poids total du pigment.

D'autres pigments diffractants sont commercialisés sous les dénominations Metalure^{®} Prismatic par la société ECKART^{®}.

D'autres structures possibles sont Fe/Al/Fe ou Al/Fe/Al, lesquelles présentent une susceptibilité magnétique on nulle.

La quantité de pigment diffractant peut varier, en poids par rapport au poids total de la première composition, par exemple de 0,1 à 5 %.

La dimension du pigment diffractant peut être comprise par exemple entre 5 et 200 µm, mieux entre 5 et 100 µm, par exemple entre 5 et 30 µm.

L'épaisseur des particules de pigment diffractant peut être inférieure ou égale à 3 µm, mieux 2 µm, par exemple de l'ordre de 1 µm.

### AUTRES AGENTS DE COLORATION

La composition peut comporter au moins un agent de coloration produisant de la lumière par absorption d'au moins une partie du spectre visible.

Un tel agent de coloration produisant une couleur par un phénomène d'absorption peut être constitué par un pigment magnétique ou non, organique ou inorganique ou hybride comportant à la fois de la matière organique et de la matière inorganique.

L'agent de coloration peut être un composé particulaire ou non.

Le cas échéant, les particules d'un même pigment magnétique constituent à la fois l'agent de coloration produisant la couleur par un phénomène d'absorption et les corps magnétiques.

Lorsque l'agent de coloration comporte un colorant, celui-ci peut être choisi parmi les colorants liposolubles et hydrosolubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent par exemple représenter de 0,1 à 20 % du poids de la première ou de la deuxième composition, voire de 0,1 à 6 %, lorsque présents.

L'agent de coloration peut encore être une laque ou un pigment organique choisi parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

L'agent de coloration peut être une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par «The Cosmetic, Toiletry, and Fragrance Association », dont le contenu est incorporé dans la présente demande par référence.

La composition peut comporter un pigment composite, comportant un noyau enrobé au moins partiellement par une écorce.

### Pigments composites

Un pigment composite peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La proportion massique du noyau peut excéder 50 % par rapport au poids total du pigment composite, par exemple aller de 50 % à 70 %, par exemple de 60 % à 70 %.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite peut être supérieure ou égale à 30, mesurée selon le protocole ci-dessous.

### Protocole de mesure de la saturation du pigment composite :

Les valeurs a* et b* dans l'espace CIE L*a*b* du pigment composite sont mesurées comme suit :
Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a*2 + b*2)^{1/2}.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 1 nm et environ 100 nm, voire entre environ 5 nm et environ 75 nm, par exemple entre environ 10 nm et environ 50 nm, notamment 20 ou 25 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut présenter un indice de réfraction supérieur ou égal à 2, voire supérieur ou égal à 2,1, par exemple supérieur ou égal à 2,2.

Le noyau inorganique peut être réalisé dans un matériau magnétique ou non choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau inorganique peut être coloré, le cas échéant.

### Matière colorante organique

La matière colorante organique peut comporter par exemple au moins un pigment organique, par exemple au moins une laque organique.

La matière colorante organique peut être choisie par exemple parmi les composés particulaires insolubles dans le milieu physiologiquement acceptable de la composition.

La matière colorante organique peut comporter par exemple des pigments, par exemple des laques organiques ou autres matières colorantes organiques, qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par «The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La teneur totale en matière colorante organique de la composition, provenant du pigment composite et d'autres pigments éventuels, peut être par exemple inférieure à 10%, par rapport au poids total de la composition.

La proportion de la matière colorante organique peut excéder 30 % par rapport au poids total du pigment composite, par exemple aller de 30 à 50 %, par exemple de 30 à 40%.

### Liant

Le liant du pigment composite peut être de tout type dès lors qu'il permet à la matière colorante organique d'adhérer à la surface du noyau inorganique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
   - les polysiloxanes modifiés (2A) comprenant au moins un radical choisi parmi, notamment, les polyéthers, les polyesters et les composés époxy (ils seront appelés « polysiloxanes modifiés »),
   - les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ₐSiX₄₋ₐ (I)

dans laquelle :
- R¹ représente C₆H₅-, (CH₃)₂ CH-CH₂- ou un radical de type C_{b} H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) : 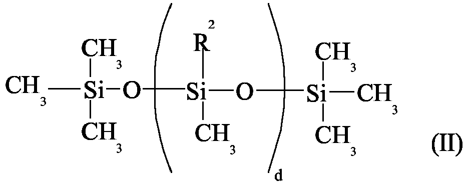 dans laquelle R² représente H- ou CH₃- et d varie de 15 à 450.

Parmi ces polysiloxanes, ceux pour lesquels R² représente H sont préférés.

Les polysiloxanes modifiés (2A) peuvent notamment répondre aux formules suivantes :
- (a¹) polysiloxanes modifiés portant des polyéthers, représentés par la formule (III) 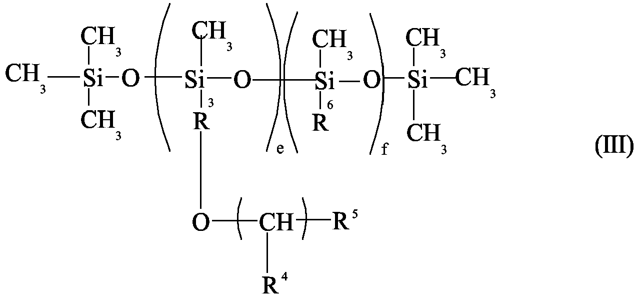 dans laquelle R³ représente -(CH₂)ₕ- ; R⁴ représente -(CH₂)ᵢ; CH₃ ; R⁵ représente -OH,-COOH, -CH = CH₂, -C (CH₃) = CH₂ ou -(CH₂)ⱼ- CH₃ ; R⁶ représente -(CH2)ₖ- CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 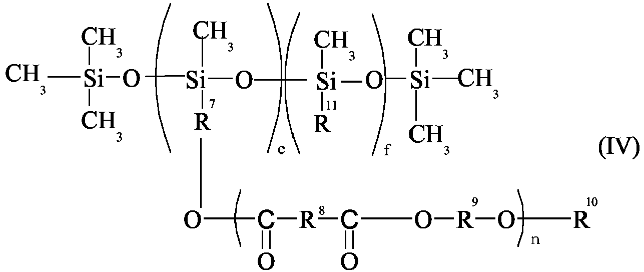 dans laquelle R⁷, R⁸ et R⁹ représentent indépendamment -(CH₂)_{q} ; R¹⁰ représente -OH ; -COOH, -CH = CH₂, -C(CH₃) = CH₂ ou -CH₂)ᵣ- CH₃ ; R¹¹ représente -(CH₂)ₛ- CH₃ ; n et q variant indépendamment de 1 à 15, r et s variant indépendamment de 0 à 15 ; e variant de 1 à 50 et f variant de 1 à 300,
- (a³) polysiloxanes modifiés portant des radicaux époxy représentés par la formule (V): 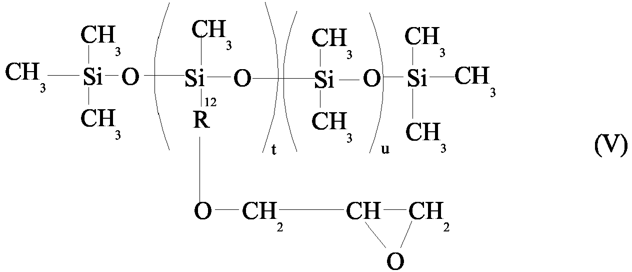 dans laquelle R¹² représente -(CH₂)ᵥ- ; v variant de 1 à 15 ; t variant de 1 à 50 et u variant de 1 à 300 ; ou leurs mélanges.
Parmi les polysiloxanes modifiés (2A), les polysiloxanes modifiés portant des polyéthers de formule (III) sont préférés.
Les polysiloxanes modifiés sur la partie terminale (2B) peuvent répondre à la formule (VI) : 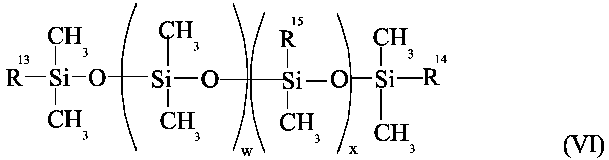 dans laquelle R¹³ et R¹⁴ peuvent représenter -OH, R¹⁶-OH ou R¹⁷-COOH, indépendamment l'un de l'autre ; R¹⁵ représente -CH₃ ou -C₆H₅ ; R¹⁶ et R¹⁷ représentent-(CH₂)y- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂(R¹⁸)ₐSiX_{4**-**a} (VII)

dans laquelle :
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)-y-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthyl-aminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2. La proportion relative de liant peut être inférieure ou égale à 5 %, par exemple inférieure ou égale à 3 %, par rapport au poids total du pigment composite.

### Préparation du pigment composite

Le pigment composite peut être préparé par tout procédé approprié, par exemple un procédé méchano-chimique ou un procédé de précipitation en solution, avec dissolution de la matière colorante organique puis précipitation à la surface du noyau.

Un liant peut être utilisé ou non.

Un procédé comportant un mélange mécanique d'un pigment organique et du noyau inorganique est préféré.

Un liant peut être ajouté et mélangé au noyau inorganique avant l'introduction de la matière colorante organique.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

Outre un agent de coloration absorbant la lumière par un phénomène d'absorption, la composition peut comporter au moins un pigment interférentiel ou diffractant, et/ou des particules réfléchissantes.

Dans un exemple de mise en oeuvre de l'invention, la première composition comporte au moins un agent de coloration goniochromatique qui permet d'observer un changement de couleur en fonction de l'angle d'observation. Cet agent de coloration goniochromatique peut être ou non magnétique.

Lorsque la première composition comporte des particules magnétiques d'une certaine couleur et un agent de coloration goniochromatique non magnétique, ce dernier peut être choisi de manière à ce que le trajet de couleur passe sensiblement par la couleur des particules magnétiques.

Cela peut permettre par exemple de rendre plus difficilement détectables les particules magnétiques à défaut d'une orientation de celles-ci sous l'effet d'un champ magnétique.

Cela peut permettre également de ne faire apparaître le motif induit par l'orientation des particules magnétiques que dans certaines conditions d'observation et/ou d'éclairage du support maquillé, ce qui peut permettre de créer des effets particulièrement attractifs d'apparition et de disposition du motif.

### Agents de coloration goniochromatiques

La composition contenant les corps magnétiques peut contenir au moins un agent de coloration goniochromatique, lequel peut présenter des propriétés magnétiques, le cas échéant.

Par « agent de coloration goniochromatique », on désigne au sens de la présente invention un agent de coloration permettant d'obtenir, lorsque la composition est étalée sur un support, un trajet de couleur dans le plan a*b* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la première composition a été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques sont par exemple les structures suivantes : Fe₂O₃/SiO₂/Fe₂O₃/SiO₂/Fe₂O₃, un pigment ayant cette structure étant commercialisé sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE^{®} HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

Le matériau peut en outre comporter des fibres goniochromatiques dispersées. De telles fibres pourront présenter une longueur inférieure à 80 µm par exemple.

### Particules réfléchissantes

La composition contenant les corps magnétiques peut comprendre des particules réfléchissantes, notamment des paillettes, entre autres, magnétiques ou non.

Par « particules réfléchissantes », on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la première composition à une teneur allant de 0,5 % à 60 % par rapport au poids total de la première composition, notamment de 1 % à 30 % en poids, en particulier de 3 % à 10 % en poids.

Ces particules peuvent présenter des formes variées, notamment être en forme de plaquettes ou globulaires, en particulier sphériques.

Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique.

Des particules réfléchissantes sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un matériau métallique, notamment un oxyde métallique, choisi par exemple parmi les oxydes de titane, notamment TiO₂, de fer notamment Fe₂O₃, d'étain, de chrome, le sulfate de baryum et les matériaux suivants : MgF₂, CrF₃, ZnS, ZnSe, SiO₂, Al₂O₃, MgO, Y₂O3, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

La composition contenant les corps magnétiques peut comprendre au moins une nacre, magnétique ou non.

### Nacres

Par « nacre », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la première composition, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

### AUTRES COMPOSANTS

Typiquement, la composition contenant les corps magnétiques comporte un milieu physiologiquement acceptable. Par « milieu physiologiquement acceptable », on désigne un milieu non toxique et susceptible d'être appliqué sur la peau, les phanères ou les lèvres d'êtres humains. Le milieu physiologiquement acceptable est généralement adapté à la nature du support sur lequel doit être appliquée la composition ainsi qu'à la forme sous laquelle la composition est conditionnée.

La composition peut comporter d'autres ingrédients que ceux décrits plus haut, notamment au moins un solvant, une phase grasse, un polymère filmogène et/ou un actif dermatologique ou cosmétique, notamment en fonction de la forme galénique.

### Solvants

La composition contenant les corps magnétiques peut comporter au moins un solvant aqueux ou organique, notamment un solvant volatil organique.

La première composition peut avantageusement comporter un solvant volatil, notamment un solvant organique volatil.

Au sens de la présente invention, on entend par « solvant volatil», un solvant, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Lorsque la composition comprend un ou plusieurs solvants organiques, ces solvants peuvent être présents en une teneur allant de 0,1 % à 99 %, par rapport au poids total de la composition concernée.

D'une manière générale, la quantité de solvant(s), notamment organique(s), dépendra de la nature du support sur lequel la composition est destinée à être appliquée.

La première composition peut comporter au moins un solvant volatil constitué par une huile volatile.

L'huile peut être une huile siliconée ou une huile hydrocarbonée, ou comporter un mélange de telles huiles.

Au sens de la présente invention, on entend par « huile siliconée », une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C8-C16 (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également citer les huiles linéaires alkyltrisiloxanes volatiles de formule générale (I) : 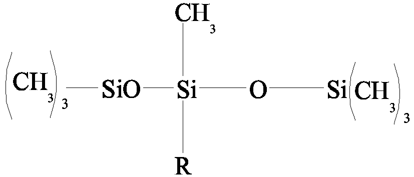 où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des huiles volatiles fluorées tels que le nonafluorométhoxybutane ou le perfluorométhylcyclopentane, et leurs mélanges.

Une composition selon l'invention peut comprendre par exemple entre 0,01 % et 95 % en poids d'huile volatile, par rapport au poids total de la composition, mieux entre 1 % et 75 % en poids.

La composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane.

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La première composition peut, en outre, contenir des éthers en C₂ et des aldéhydes en C₂-C₄ hydrophiles. L'eau ou le mélange d'eau et de solvants organiques hydrophiles peut être présent dans la première et/ou la deuxième composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La composition, par exemple lorsqu'elle est destinée à être appliquée sur les lèvres ou les cils, peut comporter une phase grasse et notamment au moins un corps gras liquide à température ambiante (25 °C) et sous pression atmosphérique (760 mm de Hg) et/ou un corps gras solide à température ambiante tel que les cires, les corps gras pâteux, les gommes et leurs mélanges. La phase grasse peut, en outre, contenir des solvants organiques lipophiles.

La composition peut présenter par exemple une phase grasse continue, pouvant contenir moins de 5 % d'eau, notamment moins de 1 % d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

La présence d'une phase huileuse peut conférer de la brillance et présenter par exemple un indice de réfraction compris entre 1,47 et 1,51, mieux entre 1,48 et 1,50. L'indice de réfraction est mesuré à température ambiante (25 °C), à l'aide d'un réfractomètre.

La composition peut comporter au moins un agent structurant de la phase grasse liquide (formée par les huiles et/ou solvants organiques volatiles ou non volatiles décrits plus haut) choisi parmi les cires, les polymères semi-cristallins, les gélifiants lipophiles et leurs mélanges.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### Polymères filmogènes

La composition peut encore comporter, par exemple, un polymère filmogène, notamment dans le cas d'un mascara, d'un vernis à ongles ou d'un fond de teint. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans la composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®,} NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

### Polymère filmogène séquencé

Selon un mode de réalisation de l'invention, la composition comprend au moins un polymère filmogène qui est un polymère éthylénique séquencé linéaire filmogène. Ce polymère comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488, qui sont incorporés par référence.

La composition contenant les corps magnétiques peut comprendre également un agent auxiliaire de filmification favorisant la formation d'un film avec le polymère filmogène.

La composition contenant les corps magnétiques peut comporter au moins une charge, magnétique ou non.

### Charges

Par « charge », on désigne des particules de toute forme, insolubles dans le milieu de la composition, quelle que soit la température à laquelle la composition est fabriquée. Une charge peut servir notamment à modifier la rhéologie ou la texture de la composition. La nature et la quantité des particules pourra dépendre des propriétés mécaniques et des textures recherchées.

A titre d'exemple de charges, on peut citer, entre autres, le talc, le mica, la silice, le kaolin, la séricite, les poudres de polyamide, de polyoléfine, par exemple de polyéthylène, de polytétrafluoroéthylène, de polyméthacrylate de méthyle, de polyuréthane, les poudres d'amidon et les billes de résine de silicone.

Les charges peuvent être destinées à créer, entre autres, un effet de flou, notamment dans le cas d'un fond de teint, afin de dissimuler des imperfections de la peau.

### Actifs

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...), autobronzants. Ces actifs peuvent être utilisés par exemple à des concentrations de 0 à 20 % et notamment de 0,001 à 15 % par rapport au poids total de la composition.

La composition peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, les colorants ou leurs mélanges.

La composition selon l'invention peut comprendre, selon le type d'application envisagée, les constituants classiquement utilisés dans les domaines considérés, qui sont présents en une quantité appropriée à la forme galénique souhaitée.

### Formes galéniques

La composition peut se présenter sous diverses formes, en fonction de sa destination. La composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situées à l'interface huile/eau.

La composition peut se présenter sous la forme d'une poudre, ou encore d'un gel.

### DISPOSITIFS MAGNETIQUES

Le dispositif magnétique peut comporter un aimant permanent ou un électroaimant, alimenté par exemple par au moins une pile ou un accumulateur. Dans ce dernier cas, le dispositif magnétique peut comporter un interrupteur permettant d'alimenter sélectivement l'électroaimant en électricité.

Le dispositif magnétique peut être agencé pour créer un champ magnétique dont l'orientation varie dans le temps. Lorsque le dispositif magnétique comporte un aimant, le dispositif peut par exemple comporter un moteur permettant d'entraîner en rotation l'aimant. En variante, le dispositif magnétique peut comporter plusieurs solénoïdes disposés de manière à générer, lorsqu'alimentés séquentiellement en électricité, un champ magnétique tournant.

Un champ magnétique rotatif peut permettre par exemple d'obtenir un motif présentant une symétrie de révolution, par exemple un motif donnant l'impression d'une sphère en relief.

Le ou les électroaimants peuvent être alimentés en permanence ou par intermittence, au choix de l'utilisateur. En particulier, le dispositif magnétique peut être agencé de telle sorte que le ou les électroaimants puissent ne pas être alimentés tant que le dispositif magnétique n'est pas positionné correctement près du support revêtu de la première composition.

Le champ magnétique est par exemple d'au moins 50 mT, voire d'au moins 66 mT, mieux d'au moins 0,2 T, voire d'au moins 1 T (10 000 Gauss).

De manière à rendre plus facile l'application du champ magnétique, le dispositif magnétique peut comporter un organe permettant de le positionner relativement au support sur lequel la composition a été déposée. Cela peut permettre par exemple d'éviter que le dispositif magnétique ne vienne accidentellement en contact avec la composition et/ou de centrer le motif réalisé sur la région concernée.

Dans un exemple de mise en oeuvre de l'invention, le dispositif magnétique est solidaire d'un applicateur servant à l'application de la composition cosmétique. Cela peut permettre de réduire le nombre d'objets manipulés par l'utilisateur et faciliter le maquillage.

Dans un autre exemple de mise en oeuvre de l'invention, le dispositif magnétique comporte un aimant monté à une première extrémité d'une tige dont la deuxième extrémité est reliée à un organe de préhension d'un applicateur servant à l'application de la composition cosmétique.

Le champ magnétique peut encore être exercé au moyen d'une structure magnétique, notamment souple, comportant une alternance de pôles N et S. Une telle structure peut permettre par exemple de réaliser des motifs répétitifs sur la première composition, par exemple des rayures.

### KITS POUR LA MISE EN OEUVRE DU PROCEDE

L'invention a encore pour objet, selon un autre de ses aspects, un kit pour la mise en oeuvre du procédé tel que défini plus haut, comportant :
- un dispositif magnétique permettant de générer un champ magnétique,
- un applicateur cosmétique non magnétique,
- une composition cosmétique comportant
   - des corps magnétiques présentant une susceptibilité magnétique non nulle et mobiles sous l'effet d'un champ magnétique,
   - au moins un pigment diffractant,
- le dispositif magnétique étant apte à créer un champ magnétique susceptible, lorsque les matières kératiniques recouvertes d'un dépôt de ladite composition sont introduites dans ledit champ magnétique, de modifier l'orientation et/ou la position des corps magnétiques à l'intérieur du dépôt, le champ magnétique étant appliqué de manière à former au moins un motif sur la composition.

Le dispositif magnétique peut notamment être agencé pour générer un champ magnétique suffisamment fort pour pouvoir modifier l'orientation et/ou la position des corps magnétiques au sein de la composition après application sur un support tel que la peau, les muqueuses ou les phanères, de manière à en changer l'aspect.

Le champ magnétique est par exemple exercé peu de temps après le dépôt, de manière à changer l'aspect de la composition avant séchage de celle-ci, lorsque la première composition comporte un solvant volatil.

La composition peut être par exemple un vernis à ongles, un fond de teint ou un rouge à lèvres, et présenter les caractéristiques telles que définies plus haut.

Le dispositif magnétique peut être tel que défini plus haut.

Le kit peut comporter un boîtier logeant la première composition cosmétique et le dispositif magnétique. Dans ce cas, le boîtier peut comporter par exemple une pluralité d'aimants de formes différentes pour réaliser des motifs différents.

Le kit peut comporter en outre une composition cosmétique supplémentaire à appliquer sur la composition précitée ou sur le support avant d'appliquer celle-ci.

L'invention pourra être mieux comprise à la lecture de la description détaillée qui va suivre, d'exemples non limitatifs de mise en oeuvre de celle-ci, ainsi qu'à l'examen du dessin annexé, sur lequel :
- la figure 1 représente, de manière schématique, un exemple de kit selon l'invention,
- la figure 2 représente en coupe axiale, schématique et partielle, le dispositif magnétique de la figure 1,
- les figures 3 et 4 illustrent de manière schématique l'utilisation du kit,
- la figure 5 représente un exemple de motif pouvant être obtenu grâce à l'invention,
- la figure 6 représente de manière schématique un récipient contenant une composition supplémentaire pouvant être appliquée sur le support,
- la figure 7 représente isolément, de manière schématique, un autre exemple de dispositif magnétique pouvant être utilisé,
- la figure 8 représente de manière schématique le dispositif magnétique de la figure 7 muni d'un organe de positionnement de l'aimant vis-à-vis du support maquillé,
- les figures 9 et 10 représentent de manière schématique d'autres exemples de kits selon l'invention,
- la figure 11 illustre l'utilisation du kit de la figure 10,
- la figure 12 représente en élévation et isolément, de manière schématique, un exemple d'applicateur solidaire d'un dispositif magnétique,
- la figure 13 est une coupe axiale et schématique d'un autre exemple de kit selon l'invention,
- la figure 14 représente de manière schématique un autre exemple de kit selon l'invention,
- la figure 15 représente un autre exemple de dispositif de conditionnement de la composition,
- la figure 16 représente un masque ajouré pouvant être utilisé lors de la mise en oeuvre du procédé selon l'invention, et
- la figure 17 représente une feuille magnétique pouvant être utilisée lors de la mise en oeuvre du procédé selon l'invention.

Sur les figures, des corps magnétiques sont représentés sous la forme de points dans un souci de clarté du dessin, mais dans la réalité les corps peuvent ne pas être visibles individuellement à l'oeil nu.

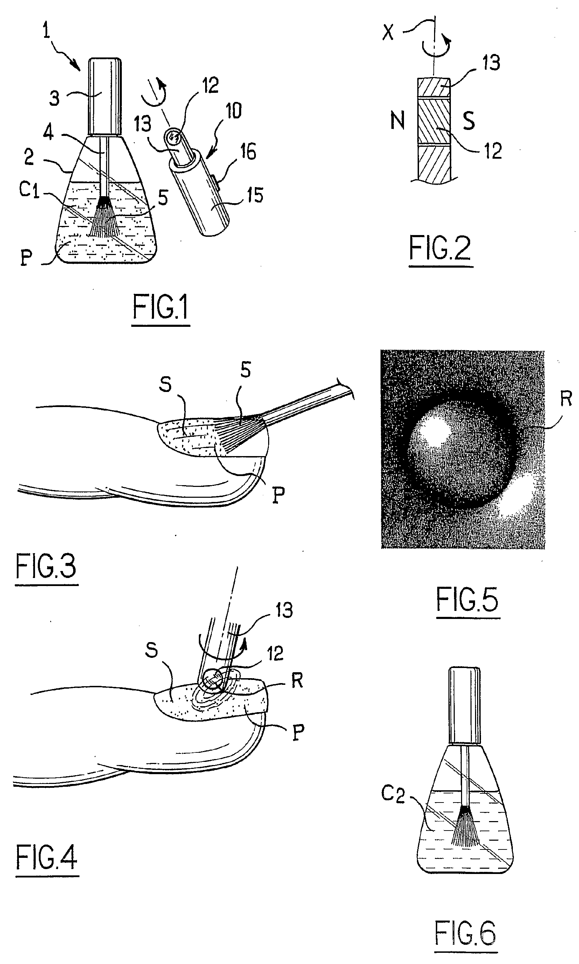

On a représenté sur la figure 1 un kit 1 comportant une composition cosmétique C₁ comprenant des particules magnétiques P dont l'orientation et/ou la position conditionnent l'aspect de la composition après dépôt sur un support tel que la peau, les muqueuses ou les phanères ou encore des faux ongles.

Dans l'exemple illustré, la composition C₁ est un vernis à ongles contenu dans un récipient 2 fermé par un capuchon 3. Ce dernier supporte un applicateur cosmétique 4, non magnétique, comportant un organe d'application 5 constitué par un pinceau permettant d'appliquer le vernis sur les ongles.

Le kit 1 comporte en outre un dispositif magnétique 10 permettant de générer un champ magnétique utile pour modifier l'aspect de la composition C₁, sans contact avec celle-ci.

Dans l'exemple considéré, le dispositif magnétique 10 comporte un aimant permanent 12 supporté par un organe de maintien 13 d'axe longitudinal X, l'axe polaire de l'aimant 12 étant sensiblement perpendiculaire à l'axe X.

Dans l'exemple considéré, le dispositif magnétique 10 est agencé pour générer un champ magnétique tournant et comporte un moteur non apparent, logé dans un boîtier 15, pour entraîner en rotation l'organe de maintien 13 autour de son axe X.

Un interrupteur 16 est présent sur le boîtier 15 pour permettre à l'utilisateur de mettre en marche le moteur et d'entraîner en rotation l'organe de maintien 13 avec l'aimant 12.

Dans une variante de réalisation non illustrée, le champ magnétique tournant est généré par une pluralité de solénoïdes alimentés séquentiellement de manière à générer un champ tournant.

Pour utiliser le kit 1, l'utilisateur peut commencer, comme illustré sur la figure 3, par appliquer sur le support à maquiller S, en l'occurrence un ongle, la composition C₁ à l'aide de l'applicateur 4.

Dans l'étape ultérieure illustrée sur la figure 4, l'utilisateur amène le dispositif magnétique 10 au-dessus d'une région centrale R du support S et actionne l'interrupteur 16 de manière à faire tourner l'aimant 12.

Les particules magnétiques contenues dans la composition C₁ tendent à s'aligner avec les lignes de champ de l'aimant 12 et changent d'orientation, ce qui entraîne une modification de l'aspect de la composition C₁.

L'utilisateur peut choisir la durée d'application du champ magnétique en fonction du résultat qu'il souhaite obtenir.

Le motif obtenu peut par exemple donner l'impression d'une sphère en relief, comme illustré sur la figure 5.

Le cas échéant, l'utilisateur peut appliquer une composition supplémentaire C₂, par exemple un vernis transparent, contenu dans un récipient représenté à la figure 6, une fois que la composition C₁ a séché.

L'application de cette deuxième composition C₂ peut permettre de créer un effet de profondeur supplémentaire, par exemple.

La composition C₁ peut avoir dans l'exemple du vernis à ongles des figures 1 à 5 la formulation suivante, les quantités étant exprimées dans tous les exemples ci-après en fractions massiques.

### Exemple A

| | |
|---|---|
| Nitrocellulose | 11 |
| N-éthyl o,p-toluènesulfonamide | 5 |
| Résine alkyde | 10 |
| Isopropanol | 4 |
| Pigment magnétiques* | 0,5 |
| Pigment diffractant** | 3 |
| Acétate de butyle/acétate d'éthyle 50/50 | Qsp 100 |

| | |
|---|---|
| * Nacres contenant au moins 14 % de Fe₃O₄ de référence COLORONA PLATINA GOLD (117288) commercialisées par la société MERCK ** Pigment SPECTRAFLAIR de la société FLEX PRODUCTS | |

L'aspect d'un tel vernis à ongles peut être modifié en appliquant un champ magnétique, avant que le vernis n'ait eu le temps de sécher.

Lorsqu'une deuxième composition C₂ est appliquée sur la première, celle-ci a par exemple la formulation suivante.

### Exemple B

| | |
|---|---|
| Nitrocellulose | 11 |
| N-éthyl o,p-toluènesulfonamide | 5 |
| Résine alkyde | 10 |
| Isopropanol | 4 |
| Acétate de butyle/acétate d'éthyle 50/50 | Qsp 100 |

Une telle composition peut permettre de créer un effet de profondeur supplémentaire.

En variante, la deuxième composition peut être destinée à créer un fond coloré, étant appliquée avant la première. La deuxième composition a alors par exemple la formulation suivante.

### Exemple C

| | |
|---|---|
| Nitrocellulose | 11 |
| N-éthyl o,p-toluènesulfonamide | 5 |
| Résine alkyde | 10 |
| Isopropanol | 4 |
| Pigment DC RED7 CI 15850 | 2 |
| Acétate de butyle/acétate d'éthyle 50/50 | Qsp 100 |

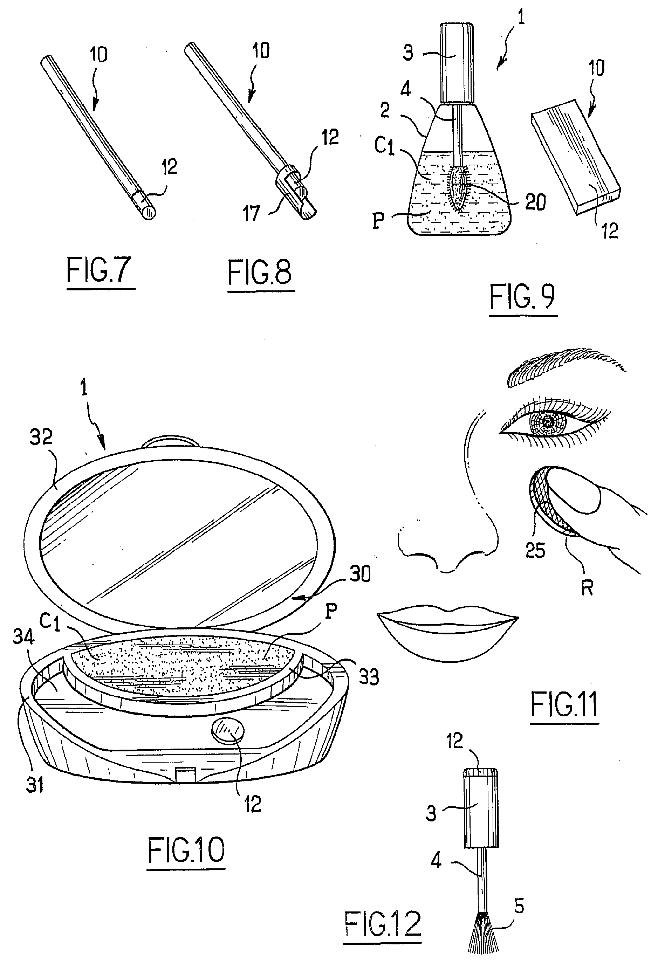

Bien entendu, le champ magnétique appliqué à la composition, quelle que soit la nature de celle-ci, peut être autre que tournant. A titre d'exemple, on a représenté à la figure 7 un dispositif magnétique qui comporte un aimant permanent 12 en forme de barreau à son extrémité.

Lorsque le champ magnétique n'est pas tournant, l'utilisateur peut par exemple déplacer l'aimant à proximité de la composition C₁ en fonction du résultat qu'il souhaite obtenir.

Le dispositif magnétique, quel qu'il soit, peut comporter un organe permettant de le positionner relativement au support S.

Cet organe de positionnement sert par exemple à éviter que le dispositif magnétique ne touche la composition pendant que le champ magnétique est exercé.

L'organe de positionnement peut servir encore à centrer le motif qui est réalisé relativement au support S, par exemple l'ongle.

Selon la nature du support, l'organe de positionnement pourra prendre diverses formes, par exemple celle d'une extension 17 offrant une surface de butée pour l'extrémité du doigt, comme illustré sur la figure 8.

On a représenté sur la figure 9 un autre exemple de kit 1 selon l'invention, comportant une première composition C₁ constituée dans cet exemple par un rouge à lèvres liquide ou un brillant à lèvres.

L'applicateur 4 comporte dans cet exemple un embout floqué 20 supporté par le capuchon 3 du récipient 2.

Le dispositif magnétique 10 se présente par exemple sous la forme d'une structure souple, par exemple en matière plastique chargée de particules aimantées, créant une alternance de pôles N et S, ce qui permet de former sur le support revêtu de la première composition des motifs répétés, par exemple des rayures.

Dans le cas d'un rouge à lèvres, la composition C₁ présente par exemple la formulation suivante.

### Exemple D

| | |
|---|---|
| Octyl-2 dodécanol | 10 |
| Ditertiobutyl 4-hydroxytoluene | 0,07 |
| Polybutène (monooléfines / isoparaffines 95/5) (PM : 2060) | 50 |
| Mélange de p-hydroxybenzoates d'isopropyle, iso-butyle, n-butyle | 0,4 |
| (40/30/30) | |
| Tetra-iso-stéarate de pentaérythrityle | 11,33 |
| Tri-mellitate de tridécyle | 13 |
| Triglycéride d'acide 2-décyl tétradécanoïque (GUERBET C24) | 15 |
| Pigment magnétique* | 0,2 |
| Pigment diffractant** | 3 |

| | |
|---|---|
| * Nacres contenant au moins 14 % de Fe₃O₄ de référence COLORONA PLATINA GOLD (117288) commercialisées par la société MERCK ** Pigment SPECTRAFLAIR de la société FLEX PRODUCTS | |

Un autre kit 1 selon l'invention a été représenté sur la figure 10. Dans cet exemple de réalisation, le kit 1 comporte un boîtier 30 constitué d'un corps de base 31 et d'un couvercle 32 articulé sur celui-ci.

Le corps de base 31 comporte un compartiment 33 logeant la composition C₁ constituée, dans l'exemple illustré, par un fond de teint sous forme pâteuse.

Le corps de base 31 comporte un logement 34 agencé pour recevoir au moins un aimant 12.

Cet aimant 12 peut présenter par exemple une face adhésive 25 ou tout autre moyen de montage permettant à l'utilisateur de le fixer à l'extrémité de son doigt de manière à l'amener à proximité de la zone maquillée, par exemple une région du visage comme illustré sur la figure 11.

Après avoir appliqué la composition C₁ sur la peau, l'utilisateur peut modifier par exemple la clarté de celle-ci en l'exposant au champ magnétique généré par l'aimant 12.

Dans le cas d'une composition de maquillage de la peau, celle-ci a par exemple la formulation suivante.

### Exemple E

| | |
|---|---|
| Sulfate de magnésium | 1,5 |
| Carboxyméthyl cellulose de sodium | 0,5 |
| Hectorite modifiée distéaryl diméthyl ammonium | 1 |
| Cyclopenta diméthylsiloxane | 15 |
| Glycérol | 5 |
| Mélange de poly-méthylcétyl diméthyl méthysiloxane oxyéthylène, iso-stéarate polyglycérol (4 moles) laurate d'hexyle | 9 |
| Eau | 30,6 |
| Mélange de stéarate d'éthylène glycol acétyle, tri-stéarate de glycéryle | 0,3 |
| Oxyde de fer brun enrobé de stéaroyl glutamate d'aluminium (97/3) | 1,58 |
| Oxyde de titane anatase enrobé de stéaroyl glutamate (97/3) | 17,17 |
| Oxyde de fer jaune enrobé de stéaroyl glutamate d'aluminium (93/3) | 4,56 |
| Oxyde de fer noir enrobé de stéaroyl glutamate d'aluminium (97/3) | 0,69 |
| Poly diméthylsiloxane (viscosité : 5 est) | 6 |
| Pigment magnétique* | 0,5 |
| Pigment diffractant** | 3,6 |
| 1,2-pentanediol | 3 |

| | |
|---|---|
| * Nacres contenant au moins 14 % de Fe₃O₄ de référence COLORONA PLATINA GOLD (117288) commercialisées par la société MERCK ** Pigment SPECTRAFLAIR de la société FLEX PRODUCTS | |

Quel que soit le type d'applicateur, l'aimant 12 peut, le cas échéant, être incorporé à l'applicateur.

Dans l'exemple de la figure 12, le capuchon 3 de fermeture est surmonté par l'aimant 12, du côté opposé à l'organe d'application 5.

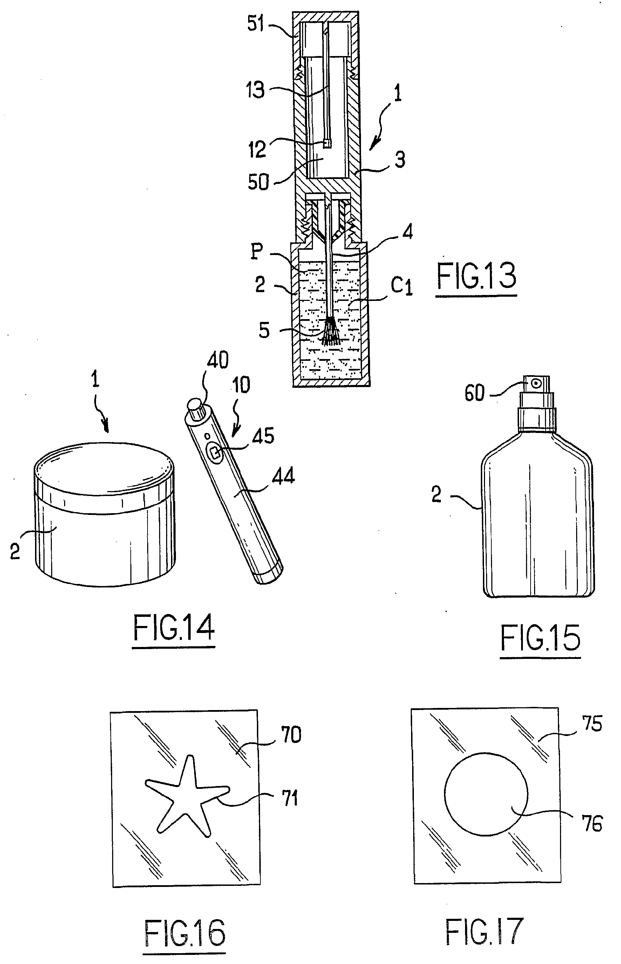

Dans l'exemple de la figure 13, l'aimant 12 est supporté par un organe de maintien 13 surmonté d'un capuchon 51 et peut se loger en l'absence d'utilisation dans un compartiment 50 du capuchon 3 permettant de fermer le récipient 2 contenant la première composition C₁. Le capuchon 51 sert à la préhension de l'aimant 12 et permet de fermer le compartiment 50.

On ne sort pas du cadre de la présente invention lorsque le champ magnétique est généré par un électroaimant au lieu d'un aimant permanent.

Sur la figure 14, on a représenté un kit 1 comportant un récipient 2 constitué par un pot contenant la première composition C₁ et un dispositif magnétique 10 comportant un électroaimant 40 à une extrémité d'un boîtier 44 logeant l'alimentation électrique.

Un interrupteur 45 permet à l'utilisateur d'alimenter sélectivement l'électroaimant 40.

Divers dispositifs autres que ceux qui viennent d'être décrits permettant de conditionner et/ou de distribuer ou appliquer la composition C₁ peuvent être utilisés.

La composition C₁ peut par exemple être déposée sur le support S sans applicateur mais sous forme de spray, par exemple en utilisant une pompe 60 comme illustré à la figure 15. Le spray peut encore être généré au moyen d'un aérographe ou d'un récipient pressurisé, par exemple.

Un masque ajouré 70, représenté sur la figure 16 et comportant comme ajour un motif 71, sous forme d'étoile dans l'exemple illustré, peut être interposé entre le spray et le support à maquiller.

Une feuille 75 perméable au champ magnétique, ajourée ou non, peut être interposée entre l'aimant 12 ou l'électroaimant 40 et le support S, de manière à modifier la géométrie des lignes de champ et créer de nouveaux effets.

Bien entendu, l'invention n'est pas limitée aux exemples qui viennent d'être décrits.

Par exemple, le kit peut comporter une pluralité d'aimants ayant des formes différentes de manière à réaliser des motifs différents.

L'expression « comportant un » doit être comprise comme étant synonyme de « comportant au moins un » et « compris entre » doit se comprendre bornes incluses, sauf si le contraire est spécifié.

## Revendications

1. Procédé de maquillage de matières kératiniques, notamment la peau, les lèvres ou les phanères, comportant les étapes suivantes :
- déposer sur le support à maquiller au moyen d'un applicateur cosmétique non magnétique au moins une composition cosmétique, cette composition cosmétique comportant :
- des corps présentant une susceptibilité magnétique non nulle et mobiles sous l'effet d'un champ magnétique,
- au moins un pigment diffractant,
- exposer au moins partiellement la composition à un champ magnétique de manière à modifier l'orientation et/ou déplacer au moins une partie des corps magnétiques,
le champ magnétique étant appliqué de manière à former au moins un motif sur la composition.

2. Procédé selon la revendication 1, dans lequel le champ magnétique est exercé par un aimant permanent.

3. Procédé selon la revendication 1, dans lequel le champ magnétique est exercé par un électroaimant.

4. Procédé selon la revendication 2 ou 3, dans lequel l'aimant ou électroaimant est entraîné en rotation.

5. Procédé selon la revendication 3, dans lequel l'électroaimant est alimenté par au moins une pile ou un accumulateur.

6. Procédé selon la revendication 3, dans lequel l'électroaimant est alimenté par intermittence lors de la formation d'un motif.

7. Procédé selon l'une quelconque des revendications précédentes, dans lequel le champ magnétique est appliqué jusqu'à obtenir un aspect figé de la composition.

8. Procédé selon l'une quelconque des revendications 1 à 6 dans lequel le champ magnétique est appliqué pendant une durée inférieure à celle provoquant l'orientation et/ou le déplacement définitif de la totalité des corps magnétiques de la région exposée.

9. Procédé selon l'une quelconque des revendications précédentes, dans lequel le champ magnétique s'exerce successivement sur différentes régions du support revêtues de la composition.

10. Procédé selon la revendication précédente, **caractérisé par le fait que** le champ magnétique s'exerce successivement sur des régions disjointes du support.

11. Procédé selon l'une quelconque des revendications précédentes, dans lequel une région au moins du support revêtue de la composition est non exposée au champ magnétique.

12. Procédé selon la revendication précédente, dans lequel l'applicateur comporte un pinceau, un embout floqué ou une mousse.

13. Procédé selon l'une quelconque des revendications précédentes, **caractérisé par le fait que** les corps magnétiques sont composés au moins partiellement par un pigment.

14. Procédé selon l'une quelconque des revendications précédentes, **caractérisée par le fait que** les corps magnétiques comportent des fibres ou chaînes de particules.

15. Procédé selon l'une quelconque des revendications précédentes, dans lequel le pigment diffractant est magnétique.

16. Procédé selon l'une quelconque des revendications 1 à 14, dans lequel le pigment diffractant est non magnétique.

17. Procédé selon l'une quelconque des revendications précédentes, dans lequel le pigment diffractant présente une structure multicouche.

18. Procédé selon l'une quelconque des revendications précédentes, dans lequel la composition comporte au moins un solvant volatil.

19. Procédé selon l'une quelconque des revendications précédentes, dans lequel la composition comporte au moins un polymère filmogène.

20. Kit de maquillage de matières kératiniques telles que la peau, les lèvres ou les phanères, comportant :
- un dispositif magnétique permettant de générer un champ magnétique,
- un applicateur cosmétique non magnétique,
- une composition cosmétique comportant
- des corps magnétiques présentant une susceptibilité magnétique non nulle et mobiles sous l'effet d'un champ magnétique,
- au moins un pigment diffractant,
- le dispositif magnétique étant apte à créer un champ magnétique susceptible, lorsque les matières kératiniques recouvertes d'un dépôt de ladite composition sont introduites dans ledit champ magnétique, de modifier l'orientation et/ou la position des corps magnétiques à l'intérieur du dépôt, le champ magnétique étant appliqué de manière à former au moins un motif sur la composition.

21. Kit selon la revendication précédente, dans lequel la composition est un vernis à ongles.

22. Kit selon la revendication 20, dans lequel la composition est un fond de teint.

23. Kit selon la revendication 20, dans lequel la composition est un produit à appliquer sur les lèvres.

24. Kit selon la revendication 20, dans lequel la composition est un produit à appliquer sur les fibres kératiniques.

25. Kit selon l'une quelconque des revendications 20 à 24, dans lequel les corps magnétiques sont constitués au moins en partie par le pigment diffractant.

26. Kit selon l'une quelconque des revendications 20 à 25, dans lequel les corps magnétiques sont différents du pigment diffractant.

27. Kit selon l'une quelconque des revendications 20 à 22, dans lequel le pigment diffractant présente une structure multicouche.

28. Kit selon l'une quelconque des revendications 20 à 27, dans lequel le dispositif magnétique comporte un aimant ou électroaimant.

29. Kit selon l'une quelconque des revendications 20 à 28, dans lequel la composition comporte au moins un solvant volatil.

30. Kit selon l'une quelconque des revendications 20 à 29, dans lequel la composition comporte au moins un polymère filmogène.

## Patentansprüche

1. Verfahren zum Schminken von keratinischen Substanzen, insbesondere Haut, Lippen oder Phaneren, umfassend die folgenden Schritte:
- Abscheiden auf dem zu schminkenden Träger mittels eines nicht-magnetischen kosmetischen Applikators mindestens einer kosmetischen Zusammensetzung, wobei diese kosmetische Zusammensetzung Folgendes umfasst:
- Körper, die eine magnetische Suszeptibilität von nicht Null aufweisen und unter dem Einfluss eines Magnetfeldes beweglich sind,
- mindestens ein Streupigment,
- Exponieren der Zusammensetzung gegenüber einem Magnetfeld mindestens teilweise derart, dass die Orientierung modifiziert und/oder mindestens ein Teil der Magnetkörper bewegt wird, wobei das Magnetfeld so angelegt wird, dass mindestens ein Muster auf der Zusammensetzung ausgebildet wird.

2. Verfahren nach Anspruch 1, wobei das Magnetfeld durch einen Permanentmagneten ausgeübt wird.

3. Verfahren nach Anspruch 1, wobei das Magnetfeld durch einen Elektromagneten ausgeübt wird.

4. Verfahren nach Anspruch 2 oder 3, wobei der Magnet oder Elektromagnet drehangetrieben wird.

5. Verfahren nach Anspruch 3, wobei der Elektromagnet durch mindestens eine Batterie oder einen Akkumulator gespeist wird.

6. Verfahren nach Anspruch 3, wobei der Elektromagnet während der Bildung eines Musters abwechselnd gespeist wird.

7. Verfahren nach einem der vorangehenden Ansprüche, wobei das Magnetfeld angelegt wird, bis ein unbewegliches Aussehen der Zusammensetzung erhalten wird.

8. Verfahren nach einem der Ansprüche 1 bis 6, wobei das Magnetfeld während einer Dauer unter derjenigen angelegt wird, die die Orientierung und/oder die endgültige Bewegung der Gesamtheit der Magnetkörper aus der exponierten Region hervorruft.

9. Verfahren nach einem der vorangehenden Ansprüche, wobei das Magnetfeld nach und nach auf verschiedene Bereiche des mit der Zusammensetzung überzogenen Trägers ausgeübt wird.

10. Verfahren nach dem vorangehenden Anspruch, **dadurch gekennzeichnet, dass** das Magnetfeld nach und nach auf getrennte Bereiche des Trägers ausgeübt wird.

11. Verfahren nach einem der vorangehenden Ansprüche, wobei mindestens eine Region des mit der Zusammensetzung überzogenen Trägers nicht dem Magnetfeld ausgesetzt wird.

12. Verfahren nach dem vorangehenden Anspruch, wobei der Applikator ein Pinsel, ein beflocktes Ende oder einen Schaum umfasst.

13. Verfahren nach einem der vorangehenden Ansprüche, **dadurch gekennzeichnet, dass** die Magnetkörper mindestens teilweise aus einem Pigment bestehen.

14. Verfahren nach einem der vorangehenden Ansprüche, **dadurch gekennzeichnet, dass** die Magnetkörper Fasern oder Ketten von Teilchen umfassen.

15. Verfahren nach einem der vorangehenden Ansprüche, wobei das Streupigment magnetisch ist.

16. Verfahren nach einem der Ansprüche 1 bis 14, wobei das Streupigment nicht magnetisch ist.

17. Verfahren nach einem der vorangehenden Ansprüche, wobei das Streupigment eine mehrschichtige Struktur umfasst.

18. Verfahren nach einem der vorangehenden Ansprüche, wobei die Zusammensetzung mindestens ein flüchtiges Lösungsmittel umfasst.

19. Verfahren nach einem der vorangehenden Ansprüche, wobei die Zusammensetzung mindestens ein filmbildendes Polymer umfasst.

20. Kit zum Schminken von keratinischen Substanzen, wie Haut, Lippen oder Phaneren, umfassend:
- eine magnetische Vorrichtung, die die Erzeugung eines Magnetfeldes ermöglicht,
- einen nicht-magnetischen kosmetischen Applikator,
- eine kosmetische Zusammensetzung umfassend:
- Magnetkörper, die eine magnetische Suszeptibilität von nicht Null aufweisen und unter der Wirkung eines Magnetfeldes beweglich sind,
- mindestens ein Streupigment,
- wobei die magnetische Vorrichtung dazu geeignet ist, ein Magnetfeld zu erzeugen, das, wenn die keratinischen Substanzen, die mit einer Abscheidung der Zusammensetzung bedeckt sind, in das Magnetfeld eingebracht werden, in der Lage ist, die Orientierung und/oder die Position der Magnetkörper im Inneren der Abscheidung zu modifizieren, wobei das Magnetfeld so angelegt wird, dass mindestens ein Muster auf der Zusammensetzung ausgebildet wird.

21. Kit nach dem vorangehenden Anspruch, wobei die Zusammensetzung ein Nagellack ist.

22. Kit nach Anspruch 20, wobei die Zusammensetzung eine Teintgrundierung ist.

23. Kit nach Anspruch 20, wobei die Zusammensetzung ein Produkt zum Auftragen auf die Lippen ist.

24. Kit nach Anspruch 20, wobei die Zusammensetzung ein Produkt zum Auftragen auf die keratinischen Fasern ist.

25. Kit nach einem der Ansprüche 20 bis 24, wobei die Magnetkörper mindestens zum Teil aus dem Streupigment bestehen.

26. Kit nach einem der Ansprüche 20 bis 25, wobei die Magnetkörper von dem Streupigment verschieden sind.

27. Kit nach einem der Ansprüche 20 bis 22, wobei das Streupigment eine mehrschichtige Struktur aufweist.

28. Kit nach einem der Ansprüche 20 bis 27, wobei die magnetische Vorrichtung einen Permanentmagneten oder einen Elektromagneten umfasst.

29. Kit nach einem der Ansprüche 20 bis 28, wobei die Zusammensetzung mindestens ein flüchtiges Lösungsmittel umfasst.

30. Kit nach einem der Ansprüche 20 bis 29, wobei die Zusammensetzung mindestens ein filmbildendes Polymer umfasst.

## Claims

1. A method for applying makeup to keratinous material, in particular the skin, lips or dermoskeleton, including the following steps:
- depositing, on the medium to be made up via a non-magnetic cosmetic applicator, at least one cosmetic composition, said cosmetic composition including:
- bodies having a non-null magnetic susceptibility and that are mobile under the effect of a magnetic field,
- at least one diffractive pigment,
- at least partially exposing the composition to a magnetic field so as to modify the orientation and/or move at least part of the magnetic bodies,
the magnetic field being applied so as to form at least one pattern on the composition.

2. The method according to claim 1, wherein the magnetic field is exerted by a permanent magnet.

3. The method according to claim 1, wherein the magnetic field is exerted by an electromagnet.

4. The method according to claim 2 or 3, wherein it the magnet or electromagnet is rotated.

5. The method according to claim 3, wherein the electromagnet is powered by at least one battery or accumulator.

6. The method according to claim 3, wherein the electromagnet is powered intermittently during the formation of a pattern.

7. The method according to any one of the preceding claims, wherein the magnetic field is applied until obtaining a frozen appearance of the composition.

8. The method according to any one of claims 1 to 6, wherein the magnetic field is applied for a duration shorter than that causing the orientation and/or the definitive movement of all of the magnetic bodies of the exposed region.

9. The method according to any one of the preceding claims, wherein the magnetic field successively exerts itself on different regions of the medium coated with the composition.

10. The method according to the preceding claims, **characterized in that** the magnetic field is exerted successively on different regions of the medium.

11. The method according to any one of the preceding claims, wherein at least one region of the medium that is coated with the composition is not exposed to the magnetic field.

12. The method according to the preceding claims, wherein the applicator includes a brush, a flocked end-piece, or a foam.

13. The method according to any one of the preceding claims, **characterized in that** the magnetic bodies are at least partially made up of a pigment.

14. The method according to any one of the preceding claims, **characterized in that** the magnetic bodies comprise fibers or particle chains.

15. The method according to any one of the preceding claims, wherein the diffracting pigment is magnetic.

16. The method according to any one of claims 1 to 14, wherein the diffracting pigment is not magnetic.

17. The method according to any one of the preceding claims, wherein the diffracting pigment has a single-layer structure.

18. The method according to any one of the preceding claims, wherein the composition includes at least one volatile solvent.

19. The method according to any one of the preceding claims, wherein the composition includes at least one film-forming agent.

20. A kit for applying makeup to keratinous material such as the skin, lips or dermoskeleton, including:
- a magnetic device making it possible to generate a magnetic field,
- a non-magnetic cosmetic applicator,
- a cosmetic composition including
- magnetic bodies having a non-null magnetic susceptibility and that are mobile under the effect of a magnetic field,
- at least one diffracting pigment,
- the magnetic device being able to create a susceptible magnetic field, when the keratinous material covered by a deposition of said composition is introduced into said magnetic field, modify the orientation and/or position of the magnetic bodies inside the deposition, the magnetic field being applied so as to form at least one pattern on the composition.

21. The kit according to the preceding claims, wherein the composition is a nail varnish.

22. The kit according to claim 20, wherein the composition is a makeup foundation.

23. The kit according to claim 20, wherein the composition is a product to be applied on the lips.

24. The kit according to claim 20, wherein the composition is a product to be applied on the keratinous fibers.

25. The kit according to any one of claims 20 to 24, wherein the magnetic bodies are made up at least in part of the diffracting pigment.

26. The kit according to any one of claims 20 to 25, wherein the magnetic bodies are different from the diffracting pigment.

27. The kit according to any one of claims 20 to 22, wherein the diffracting pigment has a multi-layer structure.

28. The kit according to any one of claims 20 to 27, wherein the magnetic device includes a magnet or an electromagnet.

29. The kit according to any one of claims 20 to 28, wherein the composition includes at least one volatile solvent.

30. The kit according to any one of claims 20 to 29, wherein the composition includes at least one film-forming agent.
